# EUROPEAN PATENT APPLICATION

(11) **EP 3 360 597 A1**
(43) Date of publication of application: **15.08.2018**
(21) Application number: 18155813.1
(22) Date of filing: 08.02.2018
(51) Int. Cl.: A61N 1/375, A61N 1/36, A61N 1/05, A61N 1/06, A61N 1/362

(54) **EXTERNAL SPINAL CORD STIMULATION DEVICES, AND ASSOCIATED SYSTEMS AND METHODS**

(30) Priority: 09.02.2017 US 201762457114 P; 05.02.2018 US 201815888874
(71) Applicant: Nevro Corp., Redwood City, CA 94065 (US)
(72) Inventor: Foreman, Bret, Redwood City, CA 94065 (US); Sunkeri, Pankaj, Redwood City, CA 94065 (US)
(74) Representative: Hoarton, Lloyd Douglas Charles

(57) **Abstract**

External spinal cord stimulation devices, and associated systems and methods. A representative system includes an external signal generator having a housing with a peripheral edge and a recess extending inwardly and along the peripheral edge. The recess can be positioned to releasably receive an elongated portion of an implantable signal delivery device. The system can further comprise electrical signal generation circuitry carried within the housing, a power source carried within the housing and coupled to the electrical signal generation circuitry, and at least one output terminal electrically coupled to the electrical signal generation circuitry and coupleable to the implantable electrical signal delivery device.

## Description

### CROSS-REFERENCE TO RELATED APPLICATION

The present application claims priority to pending U.S. Provisional Patent Application No. 62/457,114, filed on February 9, 2017, and incorporated herein by reference.

### TECHNICAL FIELD

The present technology is directed generally to external spinal cord stimulation devices, and associated systems and methods. Particular embodiments include rounded corners and/or a lead retention groove to manage the signal delivery devices that direct spinal cord stimulation signals to the patient.

### BACKGROUND

Neurological stimulators have been developed to treat pain, movement disorders, functional disorders, spasticity, cancer, cardiac disorders, and various other medical conditions. Implantable neurological stimulation systems generally have an implantable signal generator and one or more leads that deliver electrical pulses to neurological tissue or muscle tissue. For example, several neurological stimulation systems for spinal cord stimulation (SCS) have cylindrical leads that include a lead body with a circular cross-sectional shape and one or more conductive rings (i.e., contacts) spaced apart from each other at the distal end of the lead body. The conductive rings operate as individual electrodes and, in many cases, the SCS leads are implanted percutaneously through a needle inserted into the epidural space, with or without the assistance of a stylet.

Once implanted, the signal generator applies electrical pulses to the electrodes, which in turn modify the function of the patient's nervous system, such as by altering the patient's responsiveness to sensory stimuli and/or altering the patient's motor-circuit output. In SCS therapy for the treatment of pain, the signal generator applies electrical pulses to the spinal cord via the electrodes. In conventional SCS therapy, electrical pulses are used to generate sensations (known as paresthesia) that mask or otherwise alter the patient's sensation of pain. For example, in many cases, patients report paresthesia as a tingling sensation that is perceived as less uncomfortable than the underlying pain sensation. More recently, high frequency signals have been found to produce more effective pain relief, without paresthesia.

During a typical procedure, a candidate patient receives an external trial stimulator, which is worn outside the patient's body, and which is connected to a percutaneous lead that delivers a spinal cord therapy signal. Over the course of the next several days, the patient receives therapy in accordance with a variety of stimulation parameters to determine whether the patient responds, or is likely to respond, to the therapy. If the trial is successful, the patient receives a permanently implanted pulse generator. However, during the trial period, the presence of the external trial stimulator can be awkward and/or can interfere with the patient's normal activities. Accordingly, there remains a need for external stimulators that are easier and/or more convenient for the patient to use.

### BRIEF DESCRIPTION OF THE DRAWINGS

Figure 1A is a partially schematic illustration of an implantable spinal cord modulation system positioned at the spine to deliver therapeutic signals in accordance with several embodiments in the present technology.
Figure 1B is a partially schematic, cross-sectional illustration of a patient's spinal cord region, illustrating representative locations for implanted lead bodies in accordance with embodiments of the present technology.
Figure 2 is a partially schematic front view of a representative external signal generator configured in accordance with embodiments of the present technology.
Figure 3 is a partially schematic, isometric illustration of an embodiment of the external signal generator shown in Figure 2.
Figure 4 is an enlarged illustration of a portion of the external signal generator shown in Figure 2, configured in accordance with an embodiment of the present technology.
Figure 5 is an enlarged illustration of a retention element used to secure a signal delivery device to the external signal generator, in accordance with an embodiment of the present technology.
Figure 6 is a partially schematic, isometric end view of an embodiment of the external signal generator shown in Figure 2.
Figure 7 is a partially schematic illustration of a portion of a representative external signal generator configured in accordance with another embodiment of the present technology.

### DETAILED DESCRIPTION

The present technology is directed generally to external stimulators or modulators for patient treatment. Particular embodiments of the technology are directed to stimulators for delivering electrical signals to the patient's spinal cord. In other embodiments, the stimulators may be used to direct electrical signals to other regions of the patient's body, for example, the brain, heart, other organs, and/or peripheral locations. In any of these embodiments, the external stimulator can include features that are expected to increase comfort and/or convenience for the patient, practitioner, or both. For example, the external stimulator can be shaped in a manner that allows it to be easily taped to the patient's body. In addition, the external stimulator can have features that readily accommodate electrical wiring (e.g., percutaneous leads) having various lengths. This approach can reduce or eliminate the likelihood for slack portions of the lead to interfere with the patient's normal activities.

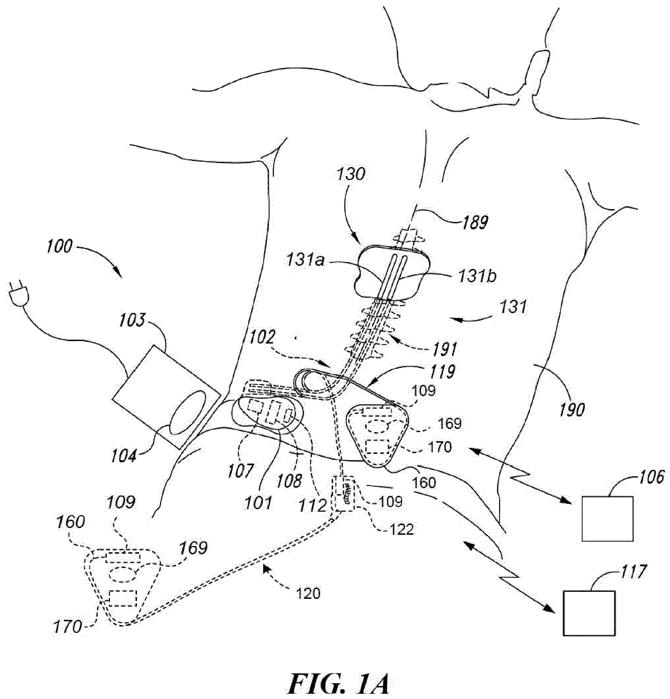

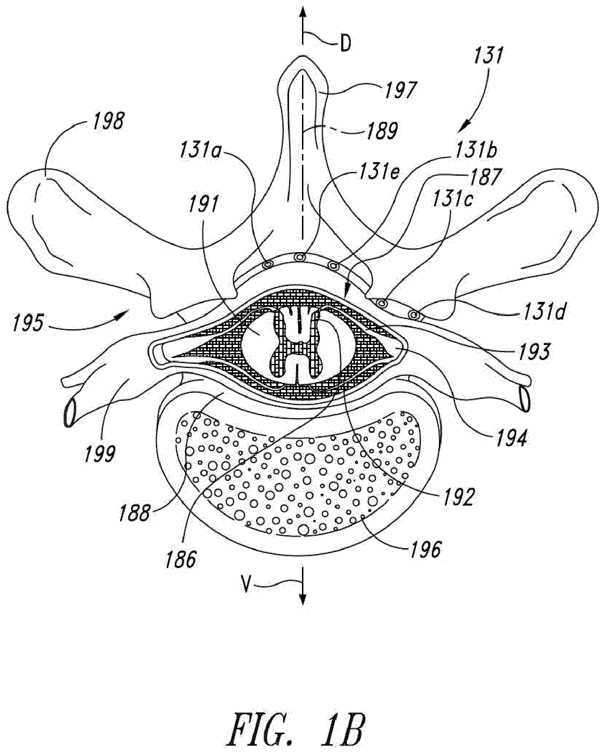

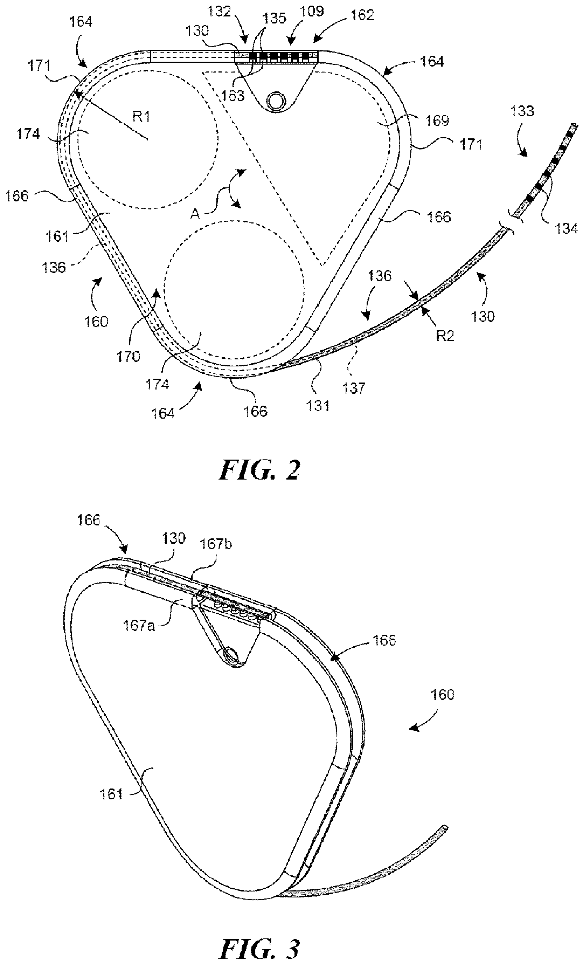

General aspects of the environments in which the disclosed technology operates are described below under Heading 1.0 ("Overview") with reference to Figures 1A and 1B. Particular embodiments of the technology along with more specific details of representative external stimulators are described further under Heading 2.0 ("Representative Embodiments") with reference to Figures 2-7. While several embodiments of the present technology are described in the environment of spinal cord stimulation (SCS), in other embodiments, one or more aspects of the present technology are applicable to other, non-SCS devices; e.g., more generally neurostimulators for treatment of one or more patient indications.

### 1.0 Overview

Figure 1A schematically illustrates a representative patient therapy system 100 (e.g., a spinal cord stimulator) for providing relief from chronic pain and/or other conditions, arranged relative to the general anatomy of a patient's spinal column 191. The system 100 can include a signal generator 101 (e.g., an implanted or implantable pulse generator or IPG), which may be implanted subcutaneously within a patient 190 and coupled to one or more signal delivery elements or devices 130. The signal delivery elements or devices 130 may be implanted within the patient 190, typically at or near the patient's spinal cord midline 189. The signal delivery elements 130 carry features for delivering therapy to the patient 190 after implantation. The signal generator 101 can be connected directly to the signal delivery devices 130, or it can be coupled to the signal delivery devices 130 via a signal link or lead extension 102. In a further representative embodiment, the signal delivery devices 130 can include one or more elongated lead(s), which can in turn include corresponding lead bodies.

As used herein, the terms signal delivery device, lead, and/or lead body include any of a number of suitable substrates and/or support members that carry electrodes/devices for providing therapy signals to the patient 190. For example, the lead or leads can include one or more electrodes or electrical contacts that direct electrical signals into the patient's tissue, e.g., to provide for therapeutic relief. In other embodiments, the signal delivery elements 130 can include structures other than a lead body (e.g., a paddle) that also direct electrical signals and/or other types of signals to the patient 190. In any of these embodiments, the lead or other signal delivery device 130, alone or in combination with other elements (e.g., the extension 102), are sometimes referred to as a lead system 119.

In a representative embodiment, one signal delivery device may be implanted on one side of the spinal cord midline 189, and a second signal delivery device may be implanted on the other side of the spinal cord midline 189. For example, first and second leads 131a, 131b shown in Figure 1A may be positioned just off the spinal cord midline 189 (e.g., about one millimeter offset) in opposing lateral directions so that the two leads 131a, 131b are spaced apart from each other by about two millimeters. In particular embodiments, the leads 131 may be implanted at a vertebral level ranging from, for example, about T8 to about T12. In other embodiments, one or more signal delivery devices can be implanted at other vertebral levels, e.g., as disclosed in U.S. Patent Application Publication No. 2013/0066411, which is incorporated herein by reference in its entirety.

The signal generator 101 can transmit signals (e.g., electrical signals) to the signal delivery elements 130 that up-regulate (e.g., excite) and/or down-regulate (e.g., block or suppress) target nerves. As used herein, and unless otherwise noted, the terms "modulate," "modulation," "stimulate," and "stimulation" refer generally to signals that have either type of the foregoing effects on the target nerves. The signal generator 101 can include a machine-readable (e.g., computer-readable or controller-readable) medium containing instructions for generating and transmitting suitable therapy signals. The signal generator 101 and/or other elements of the system 100 can include one or more processor(s) 107, memory unit(s) 108, and/or input/output device(s) 112. Accordingly, the process of providing modulation signals, providing guidance information for positioning the signal delivery devices 130, establishing battery charging and/or discharging parameters, and/or executing other associated functions can be performed by computer-executable instructions contained by, on or in computer-readable media located at the pulse generator 101 and/or other system components. Further, the signal generator 101 and/or other system components may include dedicated hardware, firmware, and/or software for executing computer-executable instructions that, when executed, perform any one or more of the methods, processes, and/or sub-processes described herein. The dedicated hardware, firmware, and/or software also serve as "means for" performing the methods, processes, and/or sub-processes described herein. The signal generator 101 can also include multiple portions, elements, and/or subsystems (e.g., for directing signals in accordance with multiple signal delivery parameters), carried in a single housing, as shown in Figure 1A, or in multiple housings.

The signal generator 101 can also receive and respond to an input signal received from one or more sources. The input signals can direct or influence the manner in which the therapy, charging, and/or process instructions are selected, executed, updated, and/or otherwise performed. The input signals can be received from one or more sensors (e.g., an input device 112 shown schematically in Figure 1A for purposes of illustration) that are carried by the signal generator 101 and/or distributed outside the signal generator 101 (e.g., at other patient locations) while still communicating with the signal generator 101. The sensors and/or other input devices 112 can provide inputs that depend on or reflect patient state (e.g., patient position, patient posture, and/or patient activity level), and/or inputs that are patient-independent (e.g., time). Still further details are included in U.S. Patent No. 8,355,797, incorporated herein by reference in its entirety.

In some embodiments, the signal generator 101 and/or signal delivery devices 130 can obtain power to generate the therapy signals from an external power source 103. In one embodiment, for example, the external power source 103 can by-pass an implanted signal generator and generate a therapy signal directly at the signal delivery devices 130 (or via signal relay components). The external power source 103 can transmit power to the implanted signal generator 101 and/or directly to the signal delivery devices 130 using electromagnetic induction (e.g., RF signals). For example, the external power source 103 can include an external coil 104 that communicates with a corresponding internal coil (not shown) within the implantable signal generator 101, signal delivery devices 130, and/or a power relay component (not shown). The external power source 103 can be portable for ease of use.

In another embodiment, the signal generator 101 can obtain the power to generate therapy signals from an internal power source, in addition to or in lieu of the external power source 103. For example, the implanted signal generator 101 can include a non-rechargeable battery or a rechargeable battery to provide such power. When the internal power source includes a rechargeable battery, the external power source 103 can be used to recharge the battery. The external power source 103 can in turn be recharged from a suitable power source (e.g., conventional wall power).

During at least some procedures, an external or trial signal generator 160 can be coupled to the signal delivery elements 130 during an initial procedure, e.g., prior to implanting the signal generator 101. For example, a practitioner (e.g., a physician and/or a company representative) can use the external signal generator 160 to vary the modulation parameters provided to the signal delivery elements 130 in real time, and select optimal or particularly efficacious parameters. These parameters can include the location from which the electrical signals are emitted, as well as the characteristics of the electrical signals provided to the signal delivery devices 130. In some embodiments, input is collected via the external signal generator 160 and can be used by the clinician to help determine what parameters to vary.

In a typical process, the practitioner uses a cable assembly 120 to temporarily connect the external signal generator 160 to the signal delivery device 130. The practitioner can test the efficacy of the signal delivery devices 130 in an initial position. The practitioner can then disconnect the cable assembly 120 (e.g., at a connector 122), reposition the signal delivery devices 130, and reapply the electrical signals. This process can be performed iteratively until the practitioner obtains the desired position for the signal delivery devices 130. Optionally, the practitioner may move the partially implanted signal delivery devices 130 without disconnecting the cable assembly 120. Furthermore, in some embodiments, the iterative process of repositioning the signal delivery devices 130 and/or varying the therapy parameters may not be performed. Instead, the practitioner can implant the signal delivery device at a particular anatomical location, and adjust the location of the electrical field by controlling which electrodes are activated

The external signal generator 160 can include signal generation circuitry 169 powered by an internal power source 170. In at least some embodiments, the signal generation circuitry 169 can include a processor and/or memory generally similar to those housed in the implantable signal generator 101. In other embodiments, the external signal generation circuitry 169 can differ from that of the implantable signal generator 101. In any of these embodiments, the external signal generation circuitry 169 can perform some or all of the functions performed by the corresponding implantable signal generator 101. The power source 170 can include one or more primary cell batteries (in which case the external pulse generator 160 is generally disposable), or a removable and replaceable battery, or a rechargeable battery, or another suitable energy source (e.g., one or more super capacitators). Further details of representative external signal generators are provided later with reference to Figures 2-7.

The implantable signal generator 101, the lead extension 102, the external signal generator 160 and/or the connector 122 can each include one or more receiving elements 109. Accordingly, the receiving elements 109 can be patient implantable elements, or the receiving elements 109 can be incorporated into an external patient treatment element, device or component (e.g., the external signal generator 160 and/or the connector 122). The receiving elements 109 can be configured to facilitate a simple coupling and decoupling procedure between the signal delivery devices 130, the lead extension 102, the implantable signal generator 101, the external signal generator 160 and/or the connector 122. In some embodiments, the receiving elements 109 can be at least generally similar in structure and function to those described in U.S. Patent Application Publication No. 2011/0071593, incorporated by reference herein in its entirety.

After the signal delivery elements 130 are implanted, the patient 190 can receive therapy via signals generated by the external signal generator 160, generally for a limited period of time. During this time, the patient can wear the cable assembly 120 and the external signal generator 160 outside the body.

In another embodiment, the cable assembly 120 can be eliminated, and instead, the external signal generator 160 can be taped directly to the patient's skin (e.g., near the entry site of the signal delivery device 130) using a suitable medical-grade adhesive, as shown in solid lines in Figure 1A. Accordingly, the external signal generator 160 can be connected directly to the signed delivery device 130 (or the extension 102) without the need for the connector 122 or the cable assembly 120, during the trial period. This approach reduces the number of components used prior to implant, thus simplifying the procedure for both the patient and the practitioner.

Assuming the trial therapy is effective or shows the promise of being effective, the practitioner then replaces the external signal generator 160 with the implantable signal generator 101, and programs the implantable signal generator 101 with therapy programs selected based on the experience gained during the trial period. Optionally, the practitioner can also replace the signal delivery element(s) 130. Once the implantable signal generator 101 has been positioned within the patient 190, the therapy programs provided by the implantable signal generator 101 can still be updated remotely via a wireless physician's programmer (e.g., a physician's laptop, a physician's remote or remote device, etc.) 117 and/or a wireless patient programmer 106 (e.g., a patient's laptop, patient's remote or remote device, etc.). Generally, the patient 190 has control over fewer parameters than does the practitioner. For example, the capability of the patient programmer 106 may be limited to starting and/or stopping the implantable signal generator 101, and/or adjusting the signal amplitude. The patient programmer 106 may be configured to accept pain relief input as well as other variables, such as medication use.

Figure 1B is a cross-sectional illustration of the spinal cord 191 and an adjacent vertebra 195 (based generally on information from Crossman and Neary, "Neuroanatomy," 1995 (published by Churchill Livingstone)), along with multiple leads 131 (shown as leads 131a-131e) implanted at representative locations. For purposes of illustration, multiple leads 131 are shown in Figure 1B implanted in a single patient. In actual use, any given patient will likely receive fewer than all the leads 131 shown in Figure 1B.

The spinal cord 191 is situated within a vertebral foramen 188, between a ventrally located ventral body 196 and a dorsally located transverse process 198 and spinous process 197. Arrows V and D identify the ventral and dorsal directions, respectively. The spinal cord 191 itself is located within the dura mater 199, which also surrounds portions of the nerves exiting the spinal cord 191, including the ventral roots 192, dorsal roots 193 and dorsal root ganglia 194. The dorsal roots 193 enter the spinal cord 191 at the dorsal root entry zone 187, and communicate with dorsal horn neurons located at the dorsal horn 186. In one embodiment, the first and second leads 131a, 131 b are positioned just off the spinal cord midline 189 (e.g., about one millimeter offset) in opposing lateral directions so that the two leads 131a, 131b are spaced apart from each other by about two millimeters, as discussed above. In other embodiments, a lead or pairs of leads can be positioned at other locations, e.g., toward the outer edge of the dorsal root entry zone 187 as shown by a third lead 131c, or at the dorsal root ganglia 194, as shown by a fourth lead 131d, or approximately at the spinal cord midline 189, as shown by a fifth lead 131e.

### 2.0 Representative Embodiments

Figure 2 is an enlarged front view of an embodiment of the external signal generator 160 described above with reference to Figure 1A. The external signal generator 160 includes a housing 161 in which the signal generation circuitry 169 and power source 170 are contained. The housing 161 is generally not configured to be implantable in the patient, which can simplify its construction and/or material requirements. In a particular embodiment, the power source 170 includes two batteries 174, e.g., two primary cell (non-rechargeable) batteries or secondary cell (rechargeable) batteries. The housing 161 also contains one or more receiver elements 109 positioned to connect the signal generation circuitry 169 with the signal delivery device 130. In this embodiment, the receiver element 109 forms a portion of a connection port 162 having a plurality of output terminals 163. The output terminals 163 are electrically connected to the signal delivery device 130, as described further below.

The signal delivery device 130 can be at least partially implantable (e.g., percutaneous or fully implantable), and can include a distal portion 133 carrying signal delivery contacts 134 that deliver electrical signals to the patient's tissue. The signal delivery device 130 can further include a proximal portion 132 carrying connection contacts 135 that connect to the output terminals 163 of the external signal generator 160. Between the distal portion 133 and the proximal portion 132, the signal delivery device 130 can include an elongated portion 136, e.g., a lead or lead body 131, which houses conductors 137. The conductors 137 connect the proximal connection contacts 135 with the corresponding distal signal delivery contacts 134. In a particular embodiment shown in Figure 2, the signal delivery device 130 includes six signal delivery contacts 134, six conductors 137, and six connection contacts 135. In other embodiments, the signal delivery device 130 can include other numbers of these elements (e.g., eight), and the external signal generator 160 can include a corresponding number of output terminals 163.

The housing 161 of the external signal generator 160 can include a peripheral edge 171 and a channel or recess 166 that extends inwardly from the peripheral edge 171. When the signal delivery device 130 is connected to the external signal generator 160 at the connection port 162, any excess length of the signal delivery device 130 (or extension 102, or other element of the lead system 119) can be wrapped around the housing 161 and pushed, inserted, and/or otherwise directed into the channel 166 where it is less likely to get caught on the patient's clothing or otherwise interfere with the patient's freedom of movement and activity. For example, this approach can prevent the signal delivery device 130 from becoming trapped between the external signal generator 160 and the patient's skin, which can cause significant discomfort for the patient. Accordingly, the corners 164 of the housing 161 are deliberately rounded with a generous radius of curvature to reduce or eliminate the likelihood for the signal delivery device 130 to become kinked or otherwise damaged when placed within the channel 166.

In one aspect of an embodiment shown in Figure 2, the signal delivery device 130 exits the channel 166 at or near one of the corners 164. For example, the signal delivery device 130 can exit the channel 166 in a direction generally tangential to the channel 166. This approach can reduce or eliminate any tendency for the signal delivery device 130 to kink or bend as it exits the channel 166.

In order to properly orient the exit direction of the signal delivery device 130 (e.g., to align with the direction in which the signal delivery device 130 enters the patient's body), the practitioner can rotate the external signal generator 160 clockwise (e.g., to take up slack in the signal delivery device 130) or counter-clockwise (e.g., to add slack to the signal delivery device 130), as indicated by arrow A. The symmetric shape of the generally triangular housing 161 provides the practitioner with flexibility to rotate the housing 161 to a suitable position.

The triangular shape of the housing 161 can provide other benefits, in addition to or in lieu of those described above. For example, the triangular shape includes a straight section at the connection port 162, where the proximal portion 132 (which can be generally rigid) of the signal delivery device 130 is oriented to provide robust physical and electrical contact with the output terminals 163. The corners 164, in addition to providing gentle curves for the wrapped section of the signal delivery device 130, can accommodate similarly curved internal elements, including one, two, or more batteries 174 (having corresponding, inwardly offset rounded edges) and the signal generator circuitry 169.

In particular embodiments, a radius R1 of the corner 164 can be directly proportional to a radius R2 of the elongated portion 136 of the signal delivery device 130. In a particular example, R1 has a value of 16.5 mm and R2 has a value of 1.5 mm, so that the ratio of R2/R1 is 11. More generally, the ratio of R2/R1 can be greater than about 10, e.g., from about 10 to 12. The ratio can vary depending on parameters such as the number of conductors 137 in the signal delivery device 130, the material(s) from which the signal delivery device 130 is formed, and/or other factors that contribute to the stiffness of the signal delivery device 130.

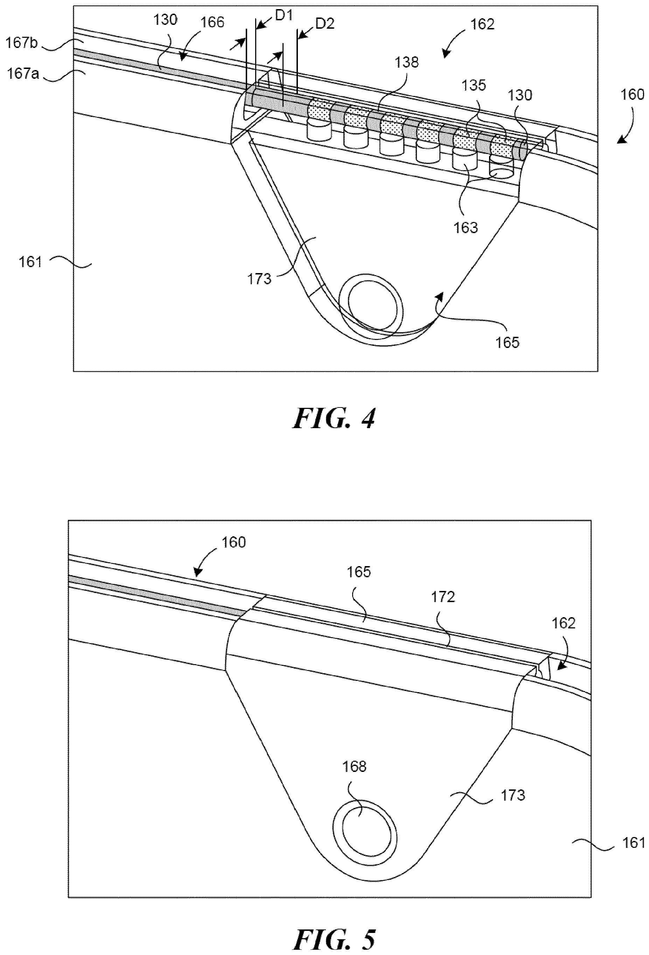

Figure 3 is an enlarged, isometric illustration of a representative external signal generator 160, illustrating the channel 166 formed in the housing 161. The channel or recess 166 can be defined, at least in part, by a first lip 167a and a second lip 167b that extend toward each other and partially close off the channel 166. As will be discussed further below with reference to Figure 4, the channel 166 and the lips 167a, 167b can be sized to releasably receive the signal delivery device 130, while restricting the signal delivery device 130 from exiting the channel 166 once placed in the channel 166, unless deliberately removed (e.g., by a practitioner).

Figure 4 is an enlarged illustration of a portion of the external signal generator 160, in particular, the region around the connection port 162. As shown in Figure 4, the first and second lips 167a, 167b can be separated from each other by a distance D1 which is less than a width or diameter D2 of the signal delivery device 130. The portion of the channel 166 positioned inwardly from the first and second lips 167a, 167b is also wider than the separation distance D1, and at least as wide as the diameter D2 of the signal delivery device 130. Accordingly, because the outer surface of the signal delivery device 130 is resilient and compressible, the practitioner can press the signal delivery device 130 to squeeze it past the lips 167a, 167b and into the channel 166, where the signal delivery device 130 will remain unless a deliberate force is applied to it to remove it from the channel 166. In particular embodiments, the lips 167a, 167b can be made of a compliant, resilient material (e.g., silicone) so as to flex out of the way as the signal delivery device 130 is inserted into the groove 166, and then flex back to the initial position to restrict or prevent the signal delivery device 130 from exiting, absent a deliberate force.

As indicated above, the signal delivery device 130, and in particular, the elongated portion 136 (Figure 3) of the signal delivery device 130, can be flexible and resilient. Accordingly, the signal delivery device 130 can conform to the patient's anatomy, as well as to the curvature of the channel 166. However, a connection region 138 of the signal delivery device 130 can be generally rigid, e.g., over a length of about 3 cm. The connection region 138 carries the connection contacts 135 so as to be in electrical contact with the corresponding output terminals 163 of the external signal generator 160. Accordingly, it is beneficial that the connection region 138 be relatively stiff and straight to better ensure physical and electrical contact between the connection contacts 135 and the corresponding output terminals 163. In particular embodiments, the output terminals 163 can include spring-loaded pins that are biased in an upward direction (in the orientation of Figure 4), so as to further enhance the electrical and physical connection between the output terminals 163 and the connection contacts 135. In a further aspect of this embodiment, the external signal generator 160 can include a retention element 165 (shown as translucent for purposes of illustration) that is positioned over the connection contacts 135 and the output terminals 163 to further facilitate a robust physical and electrical connection between these elements, and to protect the connection port 162, while also allowing deliberate access to the connection port 162, for example, if the signal delivery device 130 is to be replaced. Accordingly, the retention element 165 can include opposing lobes 173 (one of which is visible in Figure 4) that extend around the housing 161. One or both lobes 173 can include a detent or dimple 168 that is removably received in a corresponding recess of the housing 161.

Figure 5 is an enlarged illustration of the portion of the external signal generator 160 discussed above with reference to Figure 4, illustrating the retention element 165 in position over the connection port 162. To remove the retention element 165 from the housing 161, the practitioner can gently pry the lobes 173 (one of which is visible in Figure 5) away from the housing 161 so that the detent 168 exits the recess in which it is positioned, allowing the retention element 165 to be slid upwardly (as shown in Figure 5) away from the housing 161. The retention element 165 can have a groove 172 that facilitates the motion of the opposing lobes 173 away from each other as the retention element 165 is disengaged from the housing 161. In other embodiments, the retention element 165 can be releasably secured to the housing 161 using other suitable fastening arrangements. For example, the retention element 165 can have a hinged arrangement so as to remain connected to the housing 161 whether in an open or closed configuration.

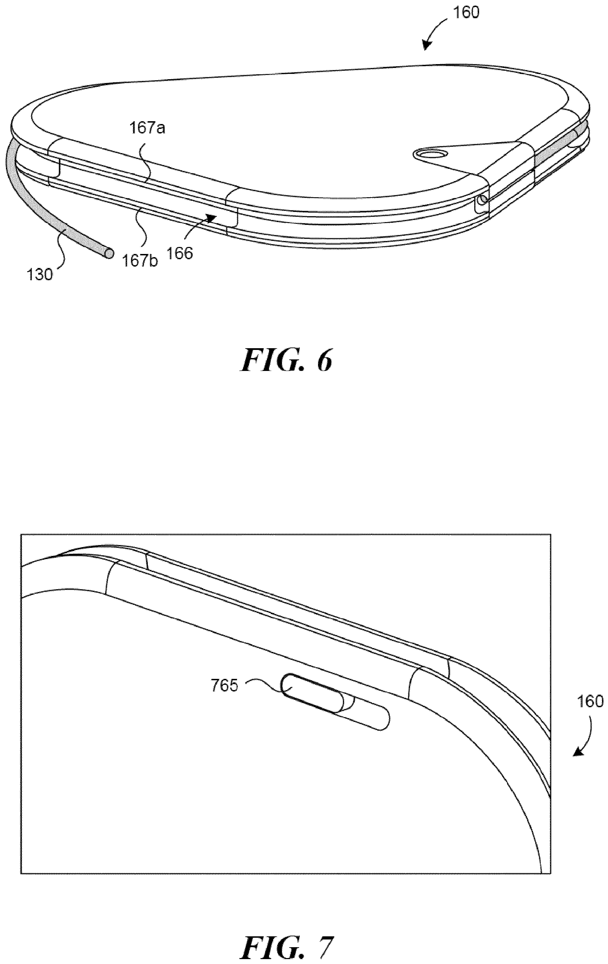

Figure 6 is a partially schematic, edge view of a representative external signal generator 160, illustrating the channel 166 and a portion of the signal delivery device 130 located partially within the channel 166. In one aspect of this embodiment, the channel 166 is sized to house two signal delivery devices 130 (one of which is shown in Figure 6), side by side. The lips 167a, 167b can be spaced apart by a distance sufficient to accommodate one signal delivery device 130 at a time, or both signal delivery devices 130 simultaneously. In either embodiment, the interior of the recess 166 accommodates two signal delivery devices simultaneously.

Figure 7 is partially schematic, isometric illustration of a portion of a representative external signal generator 160 having a retention element 765 configured in accordance with another embodiment of the present technology. In one aspect of this embodiment, the retention element 765 can include a slider that activates an internal mechanism (not visible in Figure 7) for securing the signal delivery device 130 (Figure 4) in contact with the output terminals 163 (Figure 4). In other embodiments, the external signal generator 160 can include other suitable arrangements for securing the lead 130 in electrical and physical contact with the output terminals 163.

One feature of several of the embodiments described above is that the external signal generator can have a triangular shape with gently rounded corners. The signal generator can also have a relatively small size, and can be relatively thin. These features can make the external signal generator easy and comfortable to wear when taped to the patient's skin. This arrangement can also allow the practitioner to easily wrap any excess length of the signal delivery device around the housing without causing the signal delivery device to kink, which may damage the conductors carried inside. By selecting a three-cornered arrangement, the corners can have a gentler radius than if the device where to have four or more corners. In addition, the channel or recess can capture the portion of the signal delivery device wrapped around the housing and prevent that portion from unwinding or unravelling from the housing. The foregoing features, alone or in combination, can at least restrict (e.g., prevent) the signal delivery device from interfering with the patient's motions, e.g., by catching on the patient's clothing or on nearby objects, which could be painful for the patient, and damage or dislocate the signal delivery device. This is unlike existing external stimulators, which may be larger and bulkier, and/or may be worn on the patient's belt (or on a separate belt), and/or which do not include features for stowing and/or securing the excess length or slack of the signal delivery devices.

As discussed above, the external signal generator can be used to deliver electrical signals to a patient to address a wide variety of patient indications, at a wide variety of patient locations, via one or more signal delivery devices. In a particular embodiment, the signal delivery device is placed in the patient's epidural space and is used to administer a paresthesia-free electrical signal. One example of a paresthesia-free SCS therapy system is a "high frequency" SCS system. High frequency SCS systems can inhibit, reduce, and/or eliminate pain via waveforms with high frequency elements or components (e.g., portions having high fundamental frequencies), generally with reduced or eliminated side effects. Such side effects can include unwanted paresthesia, unwanted motor stimulation or blocking, unwanted pain or discomfort, and/or interference with sensory functions other than the targeted pain. In a representative embodiment, a patient may receive high frequency therapeutic signals with at least a portion of the therapy signal at a frequency of from about 1.5 kHz to about 100 kHz, or from about 1.5 kHz to about 50 kHz, or from about 3 kHz to about 20 kHz, or from about 5 kHz to about 15 kHz, or from about 1.5 kHz to about 10 kHz, or at frequencies of about 8 kHz, 9 kHz, or 10 kHz. These frequencies are significantly higher than the frequencies associated with conventional "low frequency" SCS, which are generally below 1,200 Hz, and more commonly below 100 Hz. Accordingly, modulation at these and other representative frequencies (e.g., from about 1.5 kHz to about 100 kHz) is occasionally referred to herein as "high frequency stimulation," "high frequency SCS," and/or "high frequency modulation." Further examples of paresthesia-free SCS systems are described in U.S. Patent Publication Nos. 2009/0204173 and 2010/0274314, the respective disclosures of which are herein incorporated by reference in their entireties.

From the foregoing, it will be appreciated that specific embodiments of the disclosed technology have been described herein for purposes of illustration, but that various modifications may be made without deviating from the disclosed technology. For example, the signal delivery devices may have more or fewer than the six signal delivery contacts and corresponding connection contacts illustrated in Figures 2-7. In particular embodiments, the signal delivery device has eight signal delivery contacts. The external signal generator maybe configured to connect to and carry more than one signal delivery device (e.g., two signal delivery devices). Accordingly, the housing can include a single channel that accommodates multiple signal delivery devices placed side by side (as shown in Figure 6), or two or more parallel channels, each of which houses a corresponding signal delivery device. Or, in another embodiment, the multiple signal delivery devices can be wrapped over each other, in an accordingly deepened channel. In general, it is expected that the excess length of a given signal delivery device will be wrapped no more than once around the periphery of the house 161. In other embodiments, however, the housing can be configured to handle multiple windings of the signal delivery device. For example, the channel can be deep enough so that a signal delivery device can be wrapped multiple times around the housing, with each successive wrap overlying the one beneath. In other embodiments, the housing can include a spiral channel (e.g. wound around an axis extending out of the plane of Figure 2) to house multiple wraps of an individual signal delivery device. Multiple signal delivery devices can be wrapped multiple times in parallel channels or in a single deepened channel. The lip(s) provided at the entrance to the channel or recess can be continuous in some embodiments, or intermittent in others. For example, the lip(s) can include spaces at selected locations where the signal delivery device is expected to exit the channel.

Certain aspects of the technology described in the context of particular embodiments may be combined or eliminated in other embodiments. For example, the housing can have a three-cornered shape but need not include the channel. In such embodiments, another feature or features can be provided to restrain the motion of the signal delivery device. For example, the signal generator can include a clip that secures the signal delivery device to the housing at one or more suitable points around the periphery of the housing.

Further, while advantages associated with certain embodiments of the disclosed technology have been described in the context of those embodiments, other embodiments may also exhibit such advantages, and not all embodiments need necessarily exhibit such advantages to fall within the scope of the present technology. Accordingly, the disclosure and associated technology can encompass other embodiments not expressly shown or described herein. The following examples provide further representative embodiments of the disclosed technology.

To the extent any materials incorporated herein conflict with the present disclosure, the present disclosure controls.

### Representative features:

Clause 1. A patient therapy system, comprising:
   an external signal generator having:
      a housing with a three-cornered shape;
      electrical signal generation circuitry carried within the housing;
      a power source carried within the housing and coupled to the electrical signal generation circuitry; and
      at least one output terminal electrically coupled to the electrical signal generation circuitry and coupleable to an at least partially implantable electrical signal delivery device.
Clause 2. The system of clause 1 wherein the three corners are rounded.
Clause 3. The system of clause 1 or 2 wherein at least one of the three corners has a first radius and wherein the signal delivery device has a circular cross-sectional shape with a second radius, and wherein a ratio of the first radius to the second radius is at least 10.
Clause 4. The system of any preceding clause wherein the external housing is non-implantable.
Clause 5. The system of any preceding clause 1 wherein the external housing has a peripheral edge and includes a recess extending inwardly and along the peripheral edge, the recess being positioned to releasably receive an elongated portion of the signal delivery device.
Clause 6. The system of clause 5 wherein the recess is bounded at least in part by a first lip and a second lip, and wherein the first and second lips are separated by a distance less than a width of the elongated portion of the signal delivery device.
Clause 7. The system of clause 5 wherein the recess is bounded at least in part by a first lip and a second lip separated from the first lip by a separation distance, and wherein the system further comprises:
   the implantable electrical signal delivery device, and wherein
      the implantable electrical signal delivery device includes a lead having an elongated lead body with a diameter less than the separation distance between the first and second lips, at least a portion of the lead body being captured in the recess and restricted from exiting the recess by the first and second lips; and
      the signal delivery device includes at least one signal delivery contact connected to a corresponding at least one connection contact with a corresponding at least one conductor, the at least one connection contact being in electrical contact with the at least one output terminal.
Clause 8. The system of any preceding clause wherein at least one of the first and second lips includes a compliant resilient material.
Clause 9. The system of any preceding clause wherein at least one of the three corners is rounded, further comprising a battery having a rounded edge offset inwardly from the at least one rounded corner.
Clause 10. The system of any preceding clause, further comprising the signal delivery device.
Clause 11. The system of clause 10 wherein the signal delivery device is a percutaneous signal delivery device.
Clause 12. The system of clause 10 or 11 wherein an outer surface of the signal delivery device includes a resilient, compressible material.
Clause 13. The system of any one of clauses 10 to 12 wherein the signal delivery device has a proximal connection portion carrying connection contacts, a distal portion carrying signal delivery contacts, and an elongated portion between the proximal connection portion and the distal portion, the elongated portion carrying conductors connected between the connection contacts and the signal delivery contacts, and wherein the proximal connection portion is generally rigid.
Clause 14. The system of any preceding clause, further comprising a retention element removably carried by the housing, and movable between a first position in which it is positioned over the output terminal, and a second position in which it is removed from the housing.
Clause 15. The system of clause 14 wherein the retention element includes multiple lobes extending along corresponding opposing surfaces of the housing, and wherein at least one of the lobes includes a detent removably received in a corresponding recess of the housing.
Clause 16. A patient therapy system, comprising:
   an external signal generator having:
      a housing with a peripheral edge and a recess extending inwardly and along the peripheral edge, the recess being positioned to releasably receive an elongated portion of an at least partially implantable signal delivery device;
      electrical signal generation circuitry carried within the housing;
      a power source carried within the housing and coupled to the electrical signal generation circuitry; and
      at least one output terminal electrically coupled to the electrical signal generation circuitry and coupleable to the implantable electrical signal delivery device.
Clause 17. The system of clause 16 wherein the recess is bounded by first and second lips, and wherein at least one of the first and second lips includes a compliant resilient material.
Clause 18. The system of clause 16 or 17 wherein the recess is positioned to receive elongated portions of multiple at least partially implantable signal delivery devices.
Clause 19. The system of any one of clauses 16 to 18 wherein the recess is a first recess and the at least partially implantable signal delivery device is a first signal delivery device, and wherein the housing further has a second recess extending inwardly and along the peripheral edge, the second recess being positioned to releasably receive an elongated portion of a second signal delivery device.
Clause 20. A patient therapy system, comprising:
   an external signal generator having:
      an external, non-implantable housing with a three-cornered shape and a peripheral edge, the peripheral edge including a recess extending inwardly and along the peripheral edge, the recess being bounded at least in part by a first lip and a second lip spaced apart from the first lip by a separation distance, the housing carrying:
         electrical signal generation circuitry;
         a power source coupled to the electrical signal generation circuitry;
         a plurality of output terminals electrically coupled to the electrical signal generation circuitry; and
         a retention element coupled to the housing; and
      a signal delivery device removably coupleable to the external signal generator, the signal delivery device including a lead having an elongated lead body with a diameter less than the separation distance between the first and second lips, the signal delivery device including a plurality of signal delivery contacts connected to a corresponding plurality of connection contacts via a corresponding plurality of conductors, the connection contacts being removably coupleable to the output terminals; and
      wherein the retention element is moveable between a first position in which it secures the signal delivery device to the housing, and a second position in which the signal delivery device is removable from the housing.
Clause 21. The system of clause 20 wherein the retention element includes multiple lobes extending along corresponding opposing surfaces of the housing, and wherein at least one of the lobes includes a detent removably received in a corresponding recess of the housing.

## Claims

1. A patient therapy system, comprising:
an external signal generator having:
a housing with a three-cornered shape;
electrical signal generation circuitry carried within the housing;
a power source carried within the housing and coupled to the electrical signal generation circuitry; and
at least one output terminal electrically coupled to the electrical signal generation circuitry and coupleable to an at least partially implantable electrical signal delivery device.

2. The system of claim 1 wherein the three corners are rounded.

3. The system of claim 2 wherein at least one of the three corners has a first radius and wherein the signal delivery device has a circular cross-sectional shape with a second radius, and wherein a ratio of the first radius to the second radius is at least 10.

4. The system of claim 1 wherein the external housing is non-implantable.

5. The system of claim 1 wherein the external housing has a peripheral edge and includes a recess extending inwardly and along the peripheral edge, the recess being positioned to releasably receive an elongated portion of the signal delivery device.

6. The system of claim 5 wherein the recess is bounded at least in part by a first lip and a second lip, and wherein the first and second lips are separated by a distance less than a width of the elongated portion of the signal delivery device.

7. The system of claim 5 wherein the recess is bounded at least in part by a first lip and a second lip separated from the first lip by a separation distance, and wherein the system further comprises:
the implantable electrical signal delivery device, and wherein
the implantable electrical signal delivery device includes a lead having an elongated lead body with a diameter less than the separation distance between the first and second lips, at least a portion of the lead body being captured in the recess and restricted from exiting the recess by the first and second lips; and
the signal delivery device includes at least one signal delivery contact connected to a corresponding at least one connection contact with a corresponding at least one conductor, the at least one connection contact being in electrical contact with the at least one output terminal.

8. The system of claim 1 wherein at least one of the first and second lips includes a compliant resilient material.

9. The system of claim 1 wherein at least one of the three corners is rounded, further comprising a battery having a rounded edge offset inwardly from the at least one rounded corner.

10. The system of claim 1, further comprising the signal delivery device.

11. The system of claim 10 wherein the signal delivery device is a percutaneous signal delivery device.

12. The system of claim 10 wherein an outer surface of the signal delivery device includes a resilient, compressible material.

13. The system of claim 1, further comprising a retention element removably carried by the housing, and movable between a first position in which it is positioned over the output terminal, and a second position in which it is removed from the housing.

14. The patient therapy system of claim 1, wherein the housing is an external, non-implantable housing with a peripheral edge, the peripheral edge including a recess extending inwardly and along the peripheral edge, the recess being bounded at least in part by a first lip and a second lip spaced apart from the first lip by a separation distance, the housing carrying a retention element coupled to the housing,
wherein the system further comprises the signal delivery device, the signal delivery device being removably coupleable to the external signal generator, the signal delivery device including a lead having an elongated lead body with a diameter less than the separation distance between the first and second lips, the signal delivery device including a plurality of signal delivery contacts connected to a corresponding plurality of connection contacts via a corresponding plurality of conductors, the connection contacts being removably coupleable to the output terminals, and
wherein the retention element is moveable between a first position in which it secures the signal delivery device to the housing, and a second position in which the signal delivery device is removable from the housing.

15. The system of claim 14 wherein the retention element includes multiple lobes extending along corresponding opposing surfaces of the housing, and wherein at least one of the lobes includes a detent removably received in a corresponding recess of the housing.
